# EUROPEAN PATENT APPLICATION

(11) **EP 4 049 588 A1**
(43) Date of publication of application: **31.08.2022**
(21) Application number: 21190189.7
(22) Date of filing: 06.08.2021
(51) Int. Cl.: A61B 5/24, A61B 5/00, A61B 5/37, A61B 5/384, A61B 5/31

(54) **METHOD OF PRESERVING HUMAN MEMORY, COGNITION AND CONSCIOUSNESS**

(30) Priority: 25.02.2021 CN 202110207036
(71) Applicant: Li, Ning, Haidan District 10089 Beijing City (CN)
(72) Inventor: Li, Ning, Haidan District 10089 Beijing City (CN)
(74) Representative: Chung, Hoi Kan

(57) **Abstract**

A method of preserving human memory, cognition and consciousness is provided. The highly intelligent new human being is the product of the fusion of the human brain and the robotic brain, comprising many new features: the skeletons and joints are made of new alloy materials, the skin is made of soft silicone, the face is decorated with soft silicone on the body surface, and the joints and other parts which need to move and transform are made of high-quality alloy steel. The human-machine high intelligence chip is the fusion of the advanced intelligent human brain chip and the intelligent robot chip, comprising the intelligence and functionality of the two chips, and can continuously keep learning and make progress. Since the human brain consciousness immortal chip is a self-learning and self-improving chip, it can self-improve and self-adjust as the outside world changes.

## Description

### CROSS-REFERENCE TO RELATED APPLICATIONS

The application claims priority to Chinese patent application No. 202110207036.7, filed on February 25, 2021, the entire contents of which are incorporated herein by reference.

### TECHNICAL FIELD

The present disclosure relates to a method to preserve human memory, cognition and consciousness forever, in particular to a method of preserving human memory, cognition and consciousness.

### BACKGROUND

Immortality of mankind is not only the greatest fantasy of mankind but also the most beautiful ideal and hope in human history. Emperor Qin Shi Huang, the number one emperor of China, had sent three thousand men and girls to Japan more than two thousand years ago to search for herbs which can make human immortality. However, after more than two thousand years of history, the ideal had not been realized yet. When talking about the human immortality, it would be considered a fantasy. Until now, with the rapid development of modern technology, there is a chance to truly realize the immortal life of human consciousness. Recently, various studies in the world had proven that making the immortal life of human memory and consciousness is entirely possible and achievable.

Implanting a chip into a human brain, and then fusing injected memory with an intelligent robot to realize the thousands of years human ideal, forming a new human being.

### SUMMARY

The present disclosure aims to provide a method of preserving human memory, cognition and consciousness to solve problems raised in the above background art.

In order to achieve the above object, the present disclosure provides the following technical solutions:
the method of preserving human memory, cognition and consciousness, comprising following steps:
S1: making a human brain consciousness immortal chip by nanotechnology, the human brain consciousness immortal chip is also called learning-type high-intelligence uptake chip, including: individual consciousness and memory, cognition, emotion and intelligence;
S2: putting the human brain consciousness immortal chip in different parts of a human brain and placing the human brain consciousness immortal chip in different areas of cerebral cortex for 1 to 6 months to detect and collect more individual information; the longer the human brain consciousness immortal chip is placed in the human brain, the more accurate memory information can be captured;
S3: taking plenty of stimulation to ensure a large amount of information is obtained in a short time, including: asking a lot of questions, and the questions will stimulate memory occurrence and reproduction of an individual, so as to record more information in a shorter time;
S4: capturing various data in the memory from the human brain consciousness immortal chip;
S5: checking the memory and modify the data, correcting and repairing a missing part of the human brain and restore the human brain to a normal state; checking details of the human brain consciousness immortal chip, including individual privacy, and modifying the individual privacy according to public moral standards and with an individual consent to make a new human being more perfect, and more normal to be consistent in line with social balance and development;
S6: protecting and processing the individual privacy: allowing the individual privacy and signing privacy protection agreement to prevent individual privacy leakage and loss; deleting the individual privacy in a chip detection stage to protect various information in the human brain, and ensure the information not to be used by violent criminal groups and perpetrators;
S7: fusing extracted data with robots to form a latest man-machine fusion chip;
S8: selecting a favorite robot model, including: a man can select a woman, an elder can select a young; selecting to replace the latest man-machine fusion chip or selecting the latest man-machine fusion chip of time limit after an experience is feasible;
S9: implanting a human-machine microchip into robot brain; and
S10: testing various thinking and action abilities of the new human being, including: individual cognitive ability tests, individual emotion and personality tests, individual action ability tests, morality, axiom, and legal cognition tests, and finally, freeing the new human being to a new life and society.

As a further aspect of the present disclosure, wherein: human-machine microchip comprises a mesh structure and a multi-head high intelligent fiber contact.

As the further aspect of the present disclosure, wherein: human-machine microchip has a size of a diameter of 20 mm and a width of 6 mm.

As the further aspect of the present disclosure, wherein: human-machine microchip has a very low power consumption, and requires no power supply device, and is suitable for remote wireless charging, and can extract and capture a large amount of individual information and store the individual information.

Compared with a prior art, benefits of the present disclosure are:
the highly intelligent new human being is the product of the fusion of the human brain and a robotic brain, comprising many new features: the skeletons and joints are made of new alloy materials, the skin is made of soft silicone, the face is decorated with soft silicone on the body surface, and the joints and other parts which need to move and transform are made of high-quality alloy steel, which makes almost exactly the same parts with a human body, such as: eyes, axillary hair, sweat glands, etc.. The eyes can blink, the nose can breathe, and the ears have hearing. The highly intelligent body energy is also improved. A human-machine high intelligence chip is the fusion of the advanced intelligent human brain chip and the intelligent robot chip, comprising the intelligence and functionality of the two chips, and is a key to achieve the human immortality. The human brain consciousness immortal chip has the same functions of the human brain, and has functions of auditory, visual, olfactory, motor abilities and other aspects, and can act according to thoughts and desires. Any change from the outside world can be changed and reflected in the human brain consciousness immortal chip, and the human brain consciousness immortal chip can continuously keep learning and make progress. The human brain consciousness immortal chip is a self-learning and self-improving chip and can selfimprove and self-adjust as the outside world changes.

### DETAILED DESCRIPTION OF THE EMBODIMENTS

A technical solution of the present disclosure will be described in further details below in combination with a specific embodiment.

A method of preserving human memory, cognition and consciousness, comprising following steps:
S1: making a human brain consciousness immortal chip by nanotechnology, the human brain consciousness immortal chip is also called learning-type high-intelligence uptake chip, including: individual consciousness and memory, cognition, emotion and intelligence;
S2: putting the human brain consciousness immortal chip in different parts of a human brain and placing the human brain consciousness immortal chip in different areas of cerebral cortex for 1 to 6 months to detect and collect more individual information; the longer the human brain consciousness immortal chip is placed in the human brain, the more accurate memory information can be captured;
S3: taking plenty of stimulation to ensure a large amount of information is obtained in a short time, including: asking a lot of questions, and the questions will stimulate memory occurrence and reproduction of an individual, so as to record more information in a shorter time;
S4: capturing various data in the memory from the human brain consciousness immortal chip;
S5: checking the memory and modify the data, correcting and repairing a missing part of the human brain and restore the human brain to a normal state; checking details of the human brain consciousness immortal chip, including individual privacy, and modifying the individual privacy according to public moral standards and with an individual consent to make a new human being more perfect, and more normal to be consistent in line with social balance and development;
S6: protecting and processing the individual privacy: allowing the individual privacy and signing privacy protection agreement to prevent individual privacy leakage and loss; deleting the individual privacy in a chip detection stage to protect various information in the human brain, and ensure the information not to be used by violent criminal groups and perpetrators;
S7: fusing extracted data with robots to form a latest man-machine fusion chip;
S8: selecting a favorite robot model, including: a man can select a woman, an elder can select a young; selecting to replace the latest man-machine fusion chip or selecting the latest man-machine fusion chip of time limit after an experience is feasible;
S9: implanting a human-machine microchip into robot brain; and
S10: testing various thinking and action abilities of the new human being, including: individual cognitive ability tests, individual emotion and personality tests, individual action ability tests, morality, axiom, and legal cognition tests, and finally, freeing the new human being to a new life and society.

The working principle of the present disclosure is:
the highly intelligent new human being is the product of the fusion of the human brain and a robotic brain, comprising many new features: the skeletons and joints are made of new alloy materials, the skin is made of soft silicone, the face is decorated with soft silicone on the body surface, and the joints and other parts which need to move and transform are made of high-quality alloy steel, which makes almost exactly the same parts with a human body, such as: eyes, axillary hair, sweat glands, etc.. The eyes can blink, the nose can breathe, and the ears have hearing. The highly intelligent body energy is also improved. A human-machine high intelligence chip is the fusion of the advanced intelligent human brain chip and the intelligent robot chip, comprising the intelligence and functionality of the two chips, and is a key to achieve the human immortality. The human brain consciousness immortal chip has the same functions of the human brain, and has functions of auditory, visual, olfactory, motor abilities and other aspects, and can act according to thoughts and desires. Any change from the outside world can be changed and reflected in the human brain consciousness immortal chip, and the human brain consciousness immortal chip can continuously keep learning and make progress. The human brain consciousness immortal chip is a self-learning and self-improving chip and can selfimprove and self-adjust as the outside world changes.

The above-mentioned embodiment is a preferred embodiment of the present disclosure, and cannot be used to limit the scope of rights of the present disclosure, and various changes can be made without departing from an object of the present disclosure within a knowledge field possessed by skilled persons in the art.

## Claims

1. A method of preserving human memory, cognition and consciousness, comprising following steps:
S1: making a human brain consciousness immortal chip by nanotechnology, the human brain consciousness immortal chip is also called learning-type high-intelligence uptake chip, including: individual consciousness and memory, cognition, emotion and intelligence;
S2: putting the human brain consciousness immortal chip in different parts of a human brain and placing the human brain consciousness immortal chip in different areas of cerebral cortex for 1 to 6 months to detect and collect more individual information; the longer the human brain consciousness immortal chip is placed in the human brain, the more accurate memory information can be captured;
S3: taking plenty of stimulation to ensure a large amount of information is obtained in a short time, including: asking a lot of questions, and the questions will stimulate memory occurrence and reproduction of an individual, so as to record more information in a shorter time;
S4: capturing various data in the memory from the human brain consciousness immortal chip;
S5: checking the memory and modify the data, correcting and repairing a missing part of the human brain and restore the human brain to a normal state; checking details of the human brain consciousness immortal chip, including individual privacy, and modifying the individual privacy according to public moral standards and with an individual consent to make a new human being more perfect, and more normal to be consistent in line with social balance and development;
S6: protecting and processing the individual privacy: allowing the individual privacy and signing privacy protection agreement to prevent individual privacy leakage and loss; deleting the individual privacy in a chip detection stage to protect various information in the human brain, and ensure the information not to be used by violent criminal groups and perpetrators;
S7: fusing extracted data with robots to form a latest man-machine fusion chip;
S8: selecting a favorite robot model, including: a man can select a woman, an elder can select a young; selecting to replace the latest man-machine fusion chip or selecting the latest man-machine fusion chip of time limit after an experience is feasible;
S9: implanting a human-machine microchip into robot brain; and
S10: testing various thinking and action abilities of the new human being, including: individual cognitive ability tests, individual emotion and personality tests, individual action ability tests, morality, axiom, and legal cognition tests, and finally, freeing the new human being to a new life and society.

2. The method of preserving human memory, cognition and consciousness according to claim 1, wherein the human-machine microchip comprises a mesh structure and a multi-head high intelligent fiber contact.

3. The method of preserving human memory, cognition and consciousness according to claim 1, wherein the human-machine microchip has a size of a diameter of 20 mm and a width of 6 mm.

4. The method of preserving human memory, cognition and consciousness according to claim 1, wherein the human-machine microchip has a very low power consumption, and requires no power supply device, and is suitable for remote wireless charging, and can extract and capture a large amount of individual information and store the individual information.
